# EUROPEAN PATENT APPLICATION

(11) **EP 4 817 656 A1**
(43) Date of publication of application: **07.10.2026**
(21) Application number: 25168287.8
(22) Date of filing: 03.04.2025
(51) Int. Cl.: A61M 5/32

(54) **DISPOSABLE INJECTION NEEDLE**

(71) Applicant: Tianjin Huahong Technology Co., Ltd, Tianjin 300074 (CN)
(72) Inventor: YANG, Hao, Tianjin, 300074 (CN); ZHANG, Libo, Tianjin, 300074 (CN)
(74) Representative: Bandpay & Greuter

(57) **Abstract**

A disposable injection needle is provided. The disposable injection needle includes a needle seat, a first sheath, a second sheath, an inner core, and a spring. an accommodating space is formed between the first sheath and the needle seat, based on the movement of the second sheath into the accommodating space, the inner core rotates from a first circumferential position to a second circumferential position, and at the second circumferential position, the inner core is adapted to be abutted by the spring and move in a direction away from the accommodating space to push the second sheath; and at the second circumferential position, the second engagement structure is adapted to pass over the first engagement structure in a direction away from the accommodating space, and the first engagement structure is adapted to prevent the second engagement structure that has passed over the first engagement structure from moving further in a direction toward the inside of the accommodating space.

## Description

### TECHNICAL FIELD

An embodiment of the present disclosure relates to the field of medical instruments, in particular to a disposable injection needle.

### BACKGROUND

An injection needle for an injection pen has been widely used as a tool, and its main function is to inject drugs into patients to achieve the purpose of treatment.

At present, the common injection needle engaged with the injection pen that is in commercially available consists of a big sheath, a small sheath, a needle seat, a tube needle and dialysis paper. After the big sheath and the small sheath are removed, the needle tip of the tube needle is always exposed, and if it is used by some patients for the second time, the infection probability will increase, or the used needle is likely to accidentally injure others and cause cross infection.

### SUMMARY

In order to alleviate or solve at least one aspect or at least one point of the above problems, the present disclosure provides a disposable injection needle, which has the function of protecting the patient, can avoid injury caused by the needle tip and infection caused by repeated use, and is convenient and fast to use.

According to an embodiment of the present disclosure, a disposable injection needle is provided, including:
a needle seat provided with a needle tube extending axially therethrough;
a first sheath, wherein one end of the first sheath is connected to one end of the needle seat to form an accommodating space between the first sheath and the needle seat, and the other end of the first sheath has a first opening;
a second sheath, wherein one end of the second sheath is located in the accommodating space, and the other end of the second sheath has a second opening and protruding from the first opening; a part of the second sheath is located in the accommodating space in an axially slidable manner;
an inner core, wherein the other end of the inner core is provided with a third opening, and the needle tube axially extends through the third opening and is adapted for being exposed via the second opening; and
a spring;
wherein:
   the one end of the second sheath is engaged with the inner core by sleeving, and is adapted for retracting towards the accommodating space to expose the needle tube against an elastic force of the spring based on an external force;
   the spring is adapted for providing an elastic force to make the second sheath and the inner core move axially towards the outside of the accommodating space together;
   an inner wall of the first sheath is provided with a first engagement structure, and the second sheath is provided with a second engagement structure engaged with the first engagement structure;
   based on the movement of the second sheath into the accommodating space, the inner core rotates from a first circumferential position to a second circumferential position, and at the second circumferential position, the inner core is adapted to be abutted by the spring and move in a direction away from the accommodating space to push the second sheath; and
   at the second circumferential position, the second engagement structure is adapted to pass over the first engagement structure in a direction away from the accommodating space, and the first engagement structure is adapted to prevent the second engagement structure that has passed over the first engagement structure from moving further in a direction toward the inside of the accommodating space.

### BRIEF DESCRIPTION OF DRAWINGS

FIG. 1 is an exploded view of a three-dimensional structure of a disposable injection needle according to an embodiment of the present disclosure;
FIG. 2 is a schematic cross-sectional view of the disposable injection needle shown in FIG. 1;
FIGS. 3 and 4 are perspective views of a first sheath according to an embodiment of the present disclosure, respectively, from different perspectives;
FIGS. 5 and 6 are perspective views of a second sheath according to an embodiment of the present disclosure, respectively, from different perspectives;
FIGS. 7 and 8 are perspective views of an inner core according to an embodiment of the present disclosure, respectively, from different perspectives;
FIG. 9 is a perspective structural view of a needle seat according to an embodiment of the present disclosure;
FIG. 10 is an assembly diagram of a first sheath and a second sheath according to an embodiment of the present disclosure;
FIG. 11 is an assembly diagram of a first sheath, a second sheath and an inner core according to an embodiment of the present disclosure;
FIGS. 12 and 13 are schematic assembly diagrams of an inner core and a second sheath in an initial state according to an embodiment of the present disclosure, wherein the inner core in FIG. 12 is provided with a first protrusion, and the inner core in FIG. 13 is not provided with first protrusions;
FIG. 14 is an assembly schematic diagram of the inner core and the first sheath in an initial state according to an embodiment of the present disclosure;
FIG. 15 is an assembly schematic diagram of the inner core and the second sheath in a use state according to an embodiment of the present disclosure;
FIG. 16 is an assembly diagram of the first sheath, the second sheath and the inner core in the use state according to an embodiment of the present disclosure;
FIG. 17 is a schematic cross-sectional view of the disposable injection needle in a use state according to an embodiment of the present disclosure;
FIGS. 18 and 19 are schematic structural views of the disposable injection needle in use according to an embodiment of the present disclosure, in which the needle tube is in the longest exposed state and part of the needle tube is removed to show the internal structure;
FIGS. 20 to 23 are schematic structural views of the disposable injection needle in different states after use according to an embodiment of the present disclosure;
FIGS. 24 and 25 are schematic diagrams of structural positions between components in an installation position according to an embodiment of the present disclosure;
FIGS. 26 and 27 are schematic diagrams of structural positions among components after circumferential rotation of the inner core according to an embodiment of the present disclosure;
FIGS. 28 and 29 are schematic diagrams showing the structural positions between components after the second sheath is continuously pressed to the bottom according to an embodiment of the present disclosure;
FIGS. 30 and 31 are schematic diagrams of structural positions between components after use according to an embodiment of the present disclosure.

### Reference signs:

10, Second sheath;
102, First guide block, 104, First guide groove, 1041, Second guide surface, 105, Elastic arm, 106, Sheath guide part, 107, Second opening, 108, Second step surface;
20, First sheath;
201, First opening, 202, Second guide groove, 203, Wedge-shaped structure/convex structure, 204, Transverse groove, 205, Guide rib, 206, First guide surface, 207, Second end surface, 208, First step surface, 209, Third guide groove;
30, Inner core;
302, Second guide block, 3021, Step engagement surface, 3022, Guide engagement part, 303, First concave surface, 304, Third opening, 305, First blocking surface, 306, First end surface, 307, First protrusion;
40, Needle seat;
401, Needle seat hole, 402, Needle seat surface, 403, Transverse convex rib;
50, Spring;
60, Needle tube.

### DESCRIPTION OF EMBODIMENTS

The technical solution in the embodiment of the disclosure will be clearly and completely described below with reference to the attached drawings. Obviously, the described embodiments are only part of, not all of the embodiments of the disclosure. Based on the embodiments in this disclosure, all other embodiments obtained by those skilled in the art based on this disclosure shall belong to the protection scope of this disclosure.

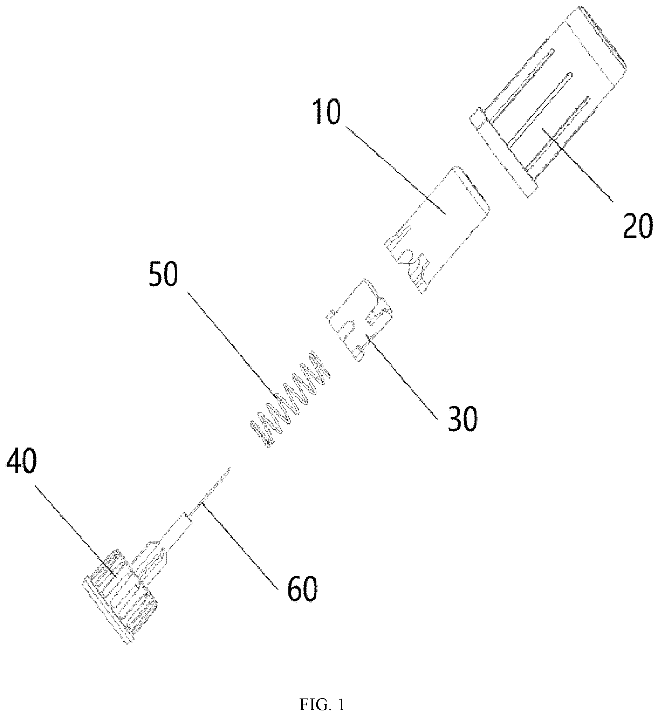

According to an exemplary embodiment of the present disclosure, as shown in FIG. 1, a disposable injection needle includes a second sheath 10, a first sheath 20, an inner core 30, a needle seat 40 and a spring 50. The needle seat 40 is provided with a needle tube 60 extending axially therethrough.

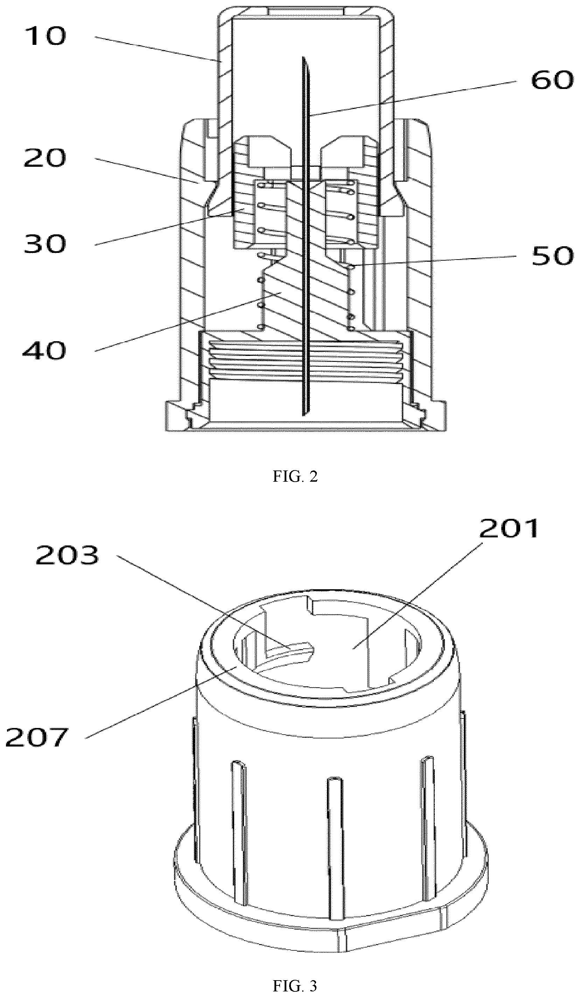

As shown in FIGS. 2, 3 and 4, one end of the first sheath 20 is connected to one end of the needle seat 40 to form an accommodation space R between the first sheath 20 and the needle seat 40, and the other end of the first sheath 20 has a first opening 201.

As shown in FIGS. 2, 5 and 6, one end of the second sheath 10 is located in the accommodation space R, the other end of the second sheath 10 has a second opening 107 and protrudes from the first opening 201, and a part of the second sheath 10 is axially slidably located in the accommodation space R.

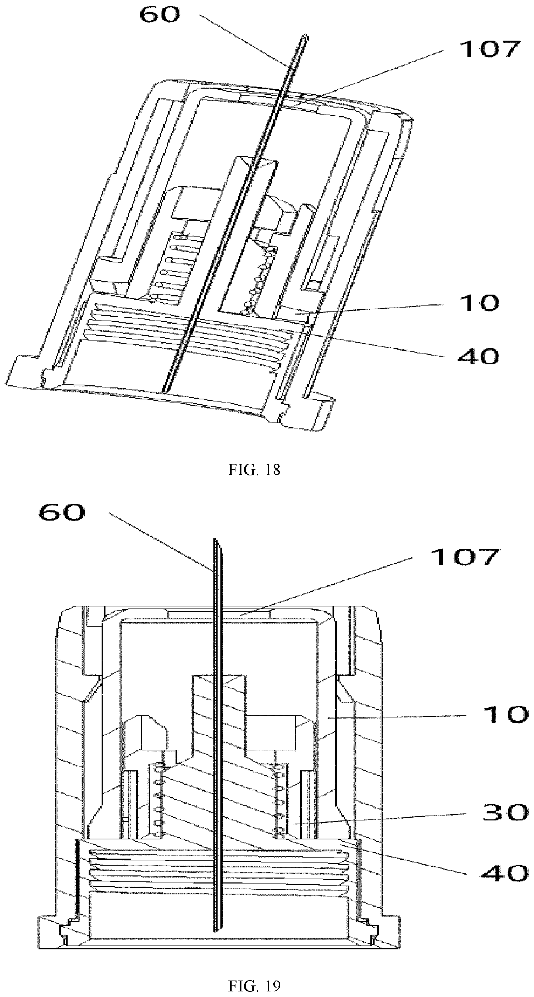

As shown in FIGS. 2, 7 and 8, the inner core 30 is arranged in the accommodating space R, and the other end of the inner core 30 has a third opening 304. The needle tube 60 extends axially through the third opening 304 and is adapted to be exposed via the second opening 107, as shown in FIGS. 18 and 19.

The spring 50 is adapted to provide an elastic force for axially moving the inner core 30 and the second sheath 10 together toward the outside of the accommodation space R. One end of the second sheath 10 is engaged with the inner core 30 by sleeving, and is adapted for retracting toward the accommodating space R against the elastic force of the spring 50 based on an external force to expose the needle tube 60, as shown in FIGS. 18 and 19.

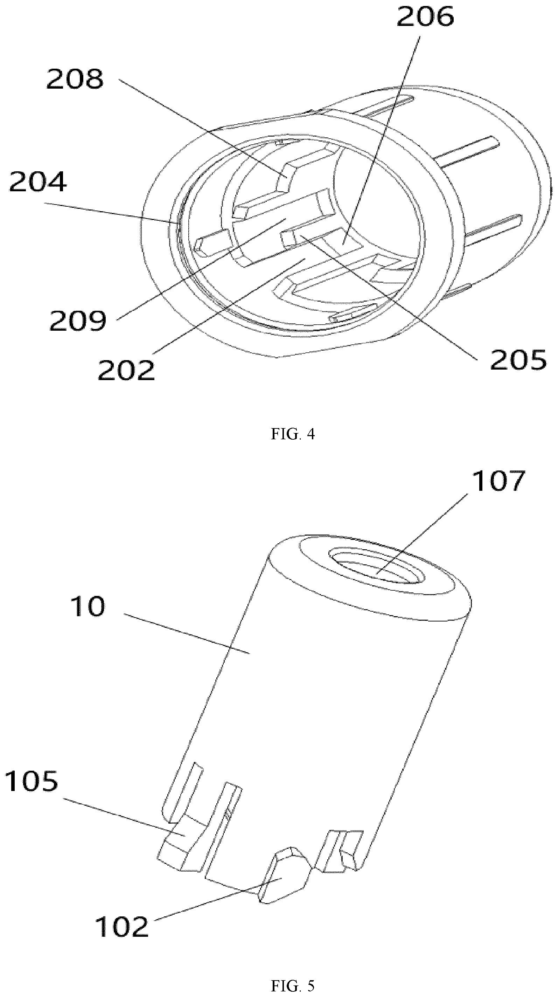

As shown in FIGS. 3, 11 and 24, the inner wall of the first sheath 20 is provided with a first engagement structure 203. As shown in FIGS. 5, 11 and 24, the second sheath 10 is provided with a second engagement structure 105 that is mated with the first engagement structure 203.

Based on the movement of the second sheath 10 into the accommodation space R, the inner core 30 rotates from a first circumferential position to a second circumferential position. At the second circumferential position, the inner core 30 is adapted to be abutted by the spring 50 and move in a direction away from the accommodation space R to push the second sheath 10.

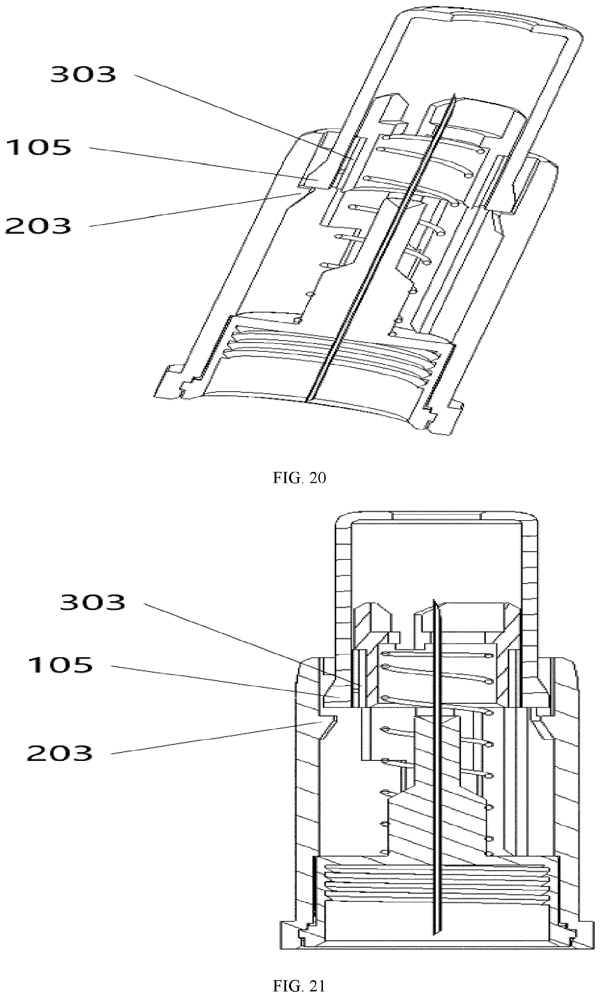

As shown in FIG. 20 and FIG. 21, at the second circumferential position, the second engagement structure 105 is adapted to pass over the first engagement structure 203 in a direction away from the accommodation space R, and the first engagement structure 203 is adapted to prevent the second engagement structure 105 that has passed over the first engagement structure 203 from moving further in a direction toward the inside of the accommodation space R.

The disposable injection needle provided by the present disclosure has the function of protecting the patient, which can avoid injuries caused by the needle tip and infection caused by repeated use, and is convenient and quick to use.

The words "up", "down", "inside" and "outside" mentioned in this disclosure are schematic descriptions. In this disclosure, the direction in which the needle tube 60 pierces or moves away from the accommodation space R is "up" and the direction in which it moves towards the accommodation space R is "down". The wall surface of the first sheath/second sheath/needle core near the central axis where the needle tube is located is "inside" and the wall surface far away from the central axis where the needle tube is located is "outside".

In an alternative embodiment, the first engagement structure is a radially inward convex structure, and the second engagement structure is an elastic arm.

In an alternative embodiment, the first engagement structure meets the following requirements: at the first circumferential position, the first engagement structure is arranged opposite to the second engagement structure, and the second engagement structure cannot be elastically deformed based on the clamping of the first engagement structure and the outer wall of the inner core; at the second circumferential position, after the second engagement structure passes over the first engagement structure, the second engagement structure can no longer move further into the accommodating space or further in the direction of the needle seat based on the blocking of the top of the first engagement structure. That is, as long as the first engagement structure can realize the function of blocking the downward movement of the second engagement structure, it shall fall into the scope of protection claimed for the first engagement structure of this disclosure.

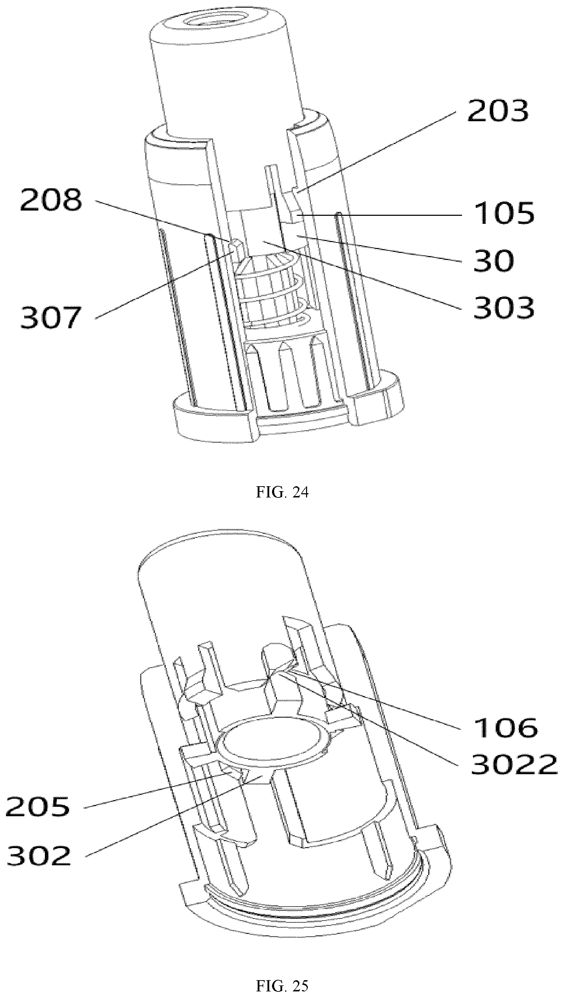

In an alternative embodiment, as shown in FIGS. 3 and 24, the radially inward protruding structure is a wedge-shaped structure 203. The wedge-shaped structure 203 is an inverted triangular pyramid structure, and the upper part thereof is a plane or a step surface.

As shown in FIGS. 5 and 24, the second engagement structure is an elastic arm 105 disposed on the second sheath 10.

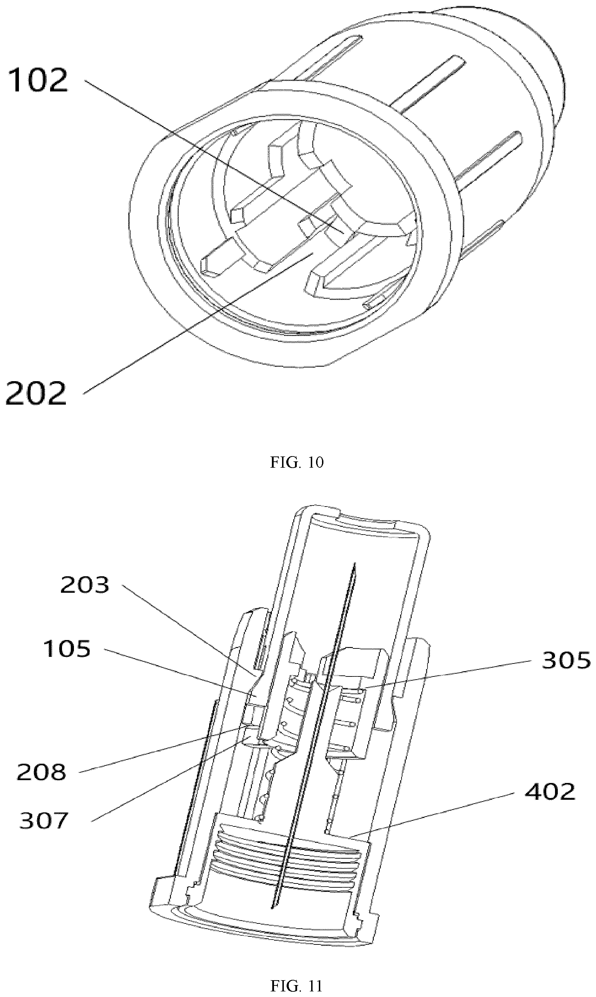

As shown in FIG. 11 and FIG. 24, at the first circumferential position, the outer side of the elastic arm 105 is opposite to the side of the wedge-shaped structure 203. At this time, the first sheath 20, the second sheath 10 and the inner core 30 are in the initial installation positions, and they are closely engaged, so that the elastic arm 105 is sandwiched between the wedge-shaped structure 203 of the first sheath 20 and the outer wall of the inner core 30, and the elastic arm 105 has no space for radial deformation.

In an alternative embodiment, at the first circumferential position, the outer side of the elastic arm 105 may or may not contact with the side of the wedge-shaped structure 203; the inner side of the elastic arm 105 is in contact with the outer side of the inner core 30, and at this time, the elastic arm 105 is naturally stretched.

In an alternative embodiment, the outer wall of the second sheath 10 is provided with a guide block extending radially outward, and the inner wall of the first sheath 20 is provided with a guide groove extending in the axial direction. The guide block is adapted for moving in the axial direction along the guide groove, so that the second sheath 10 can only move in the axial direction along the guide groove of the first sheath 20, but cannot rotate in the circumferential direction. The guide block can be arranged at any position in the circumferential direction of the first sheath 20, and correspondingly, the guide groove is arranged at any position engaged with the guide block on the inner wall of the first sheath 20.

As shown in FIG. 5, in an alternative embodiment, the guide block is a first guide block 102 extending radially outward, and the first guide block 102 is adapted for moving in the axial direction along the guide groove. The upper end of the guide groove is provided with a guide surface capable of preventing the first guide block 102 from moving upward further along the guide groove. Since the upper end of the guide groove has a guide surface, the guide surface can define the highest position that the first sheath 20 moves upward.

As shown in FIG. 4, the inner wall of the first sheath 20 is provided with a guide rib 205 extending in the axial direction, and a second guide groove 202 and a third guide groove 209 extending in the axial direction are respectively provided on the two sides of the guide rib 205.

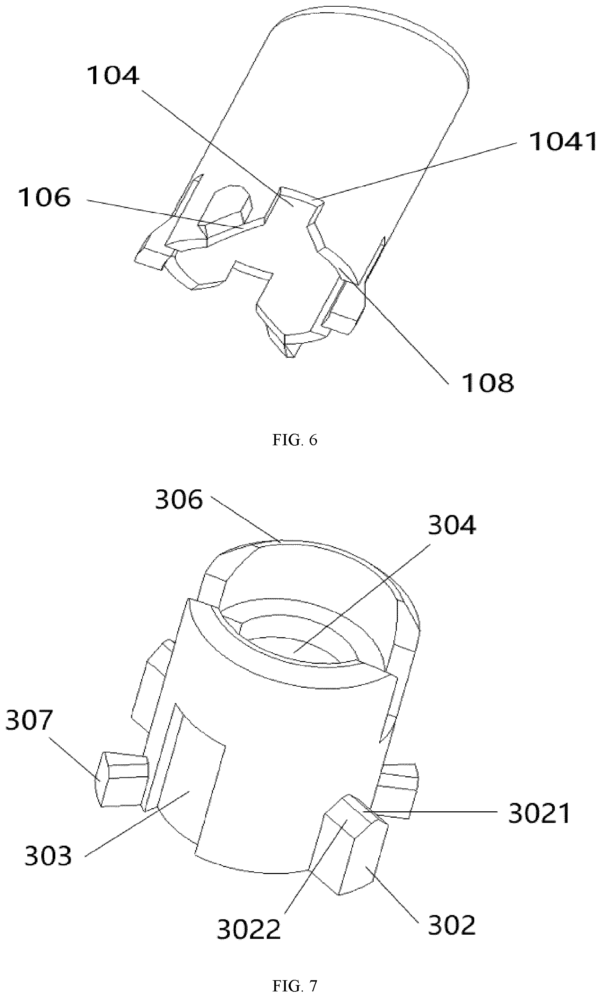

As shown in FIG. 6, one end of the second sheath 10 is provided with a sheath guide part 106, and optionally, the sheath guide part 106 is an end face of one end of the second sheath 10.

As shown in FIG. 7, one end of the inner core 30 is provided with a guide engagement part 3022. Optionally, one end of the inner core 30 is provided with a second guide block 302 extending radially outward, and the second guide block 302 has the guide engagement part 3022.

In the installation position of the disposable injection needle of the present disclosure, the guide engagement part 3022 is an inclined surface of the upper part of the second guide block 302.

Alternatively, the sheath guide part 106 and the guide engagement part 3022 are inclined planes which are engaged with each other and have the same inclination angle.

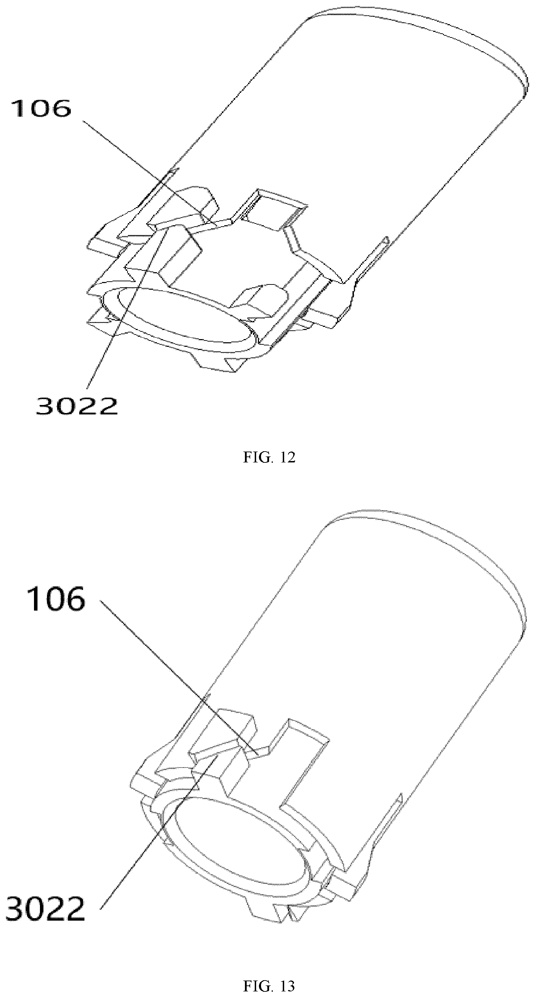

As shown in FIG. 12, FIG. 13 and FIG. 25, in the installation position of the second sheath 10 and the inner core 30, the sheath guide part 106 is engaged with the guide engagement part 3022 and is in a state of abutting contact with each other.

As shown in FIGS. 4 and 25, at this time, the second guide block 302 is located in the second guide groove 202. Because the side of the second guide block 302 is blocked by the guide rib 205, the relative sliding between the sheath guide part 106 and the guide engagement part 3022 in the circumferential direction cannot occur.

Based on the pressing on the second sheath 10 by the external force, the second sheath 10 moves toward the accommodation space R, and the inner core 30 moves downward together with the second sheath 10 until the guiding engagement part 3022 reaches the end face of the guide rib 205. At this time, the second guide block 302 is no longer blocked by the guide rib 205 in the circumferential direction, so that the guiding engagement part 3022 can move along the sheath guiding part 106 to guide the inner core 30 to rotate in the circumferential direction, and the guide engagement part 3022 is adapted to move out of engagement with the sheath guide part 106.

At this time, the second guide block 302 passes over the guide rib 205 and enters the third guide groove 209.

In an alternative embodiment, the end of the guide rib 205 has the same slope as the sheath guide part 106 and the guide engagement part 3022, so that the second guide block 302 can smoothly slide relative to the end of the guide rib 205 and pass over the guide rib 205 into the third guide groove 209.

In an alternative embodiment, the first guide block 102 on the second sheath 10 can be arranged at any position in the circumferential direction of the second sheath 10, and correspondingly, the first sheath 20 is provided with a guide groove for limiting the movement of the first guide block 102 in the axial direction.

In an alternative embodiment, as shown in FIG. 10, the guide groove is the second guide groove 202, then the first guide block 102 moves axially along the second guide groove 202. At this time, as shown in FIGS. 5 and 6, the first guide block 102 is located outside the wall where the sheath guide part 106 is located, and the end of the first guide block 102 has an inclined plane with the same inclination angle as the sheath guide part 106, so that the guide engagement part 3022 can move along the end of the sheath guide part 106 and the first guide block 102 at the same time. Thus, the first guide block 102 has the function of both making the second sheath 10 move axially and guiding the guide engagement part 3022 to rotate relatively.

As shown in FIGS. 7, 8 and 24, in an alternative embodiment, the inner core 30 has a first protrusion 307 extending radially outward. Annular ribs are formed on the inner wall of the inner core 30, a first blocking surface 305 is formed on the annular ribs, and one end of the spring 50 abuts against the first blocking surface 305.

As shown in FIGS. 4, 24 and 26, the inner wall of the first sheath 20 has a first step surface 208.

As shown in FIGS. 11, 14 and 24, the first protrusion 307 is adapted to abut against the first step surface 208 at the installation position of the first sheath 20 and the inner core 30. At this time, under the condition that no external force is applied, the first protrusion 307 abuts against the first step surface 208 based on the elastic force of the spring 50, and the second sheath and the inner core 30 remain relatively immobile and stable due to the blocking of the elastic arm 105 by the wedge-shaped structure 203.

As shown in FIG. 6, one end of the second sheath 10 has a second step surface 108.

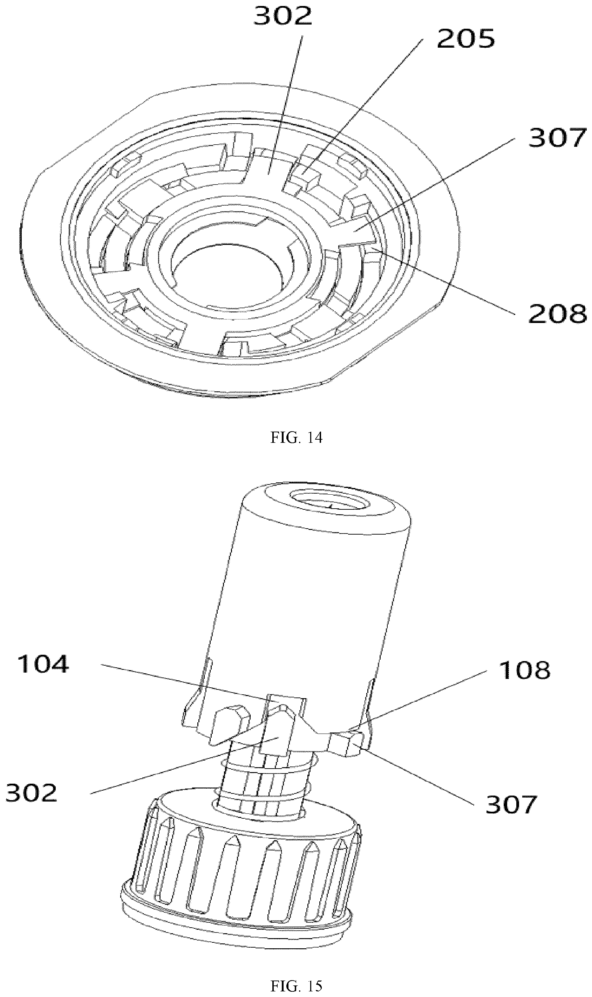

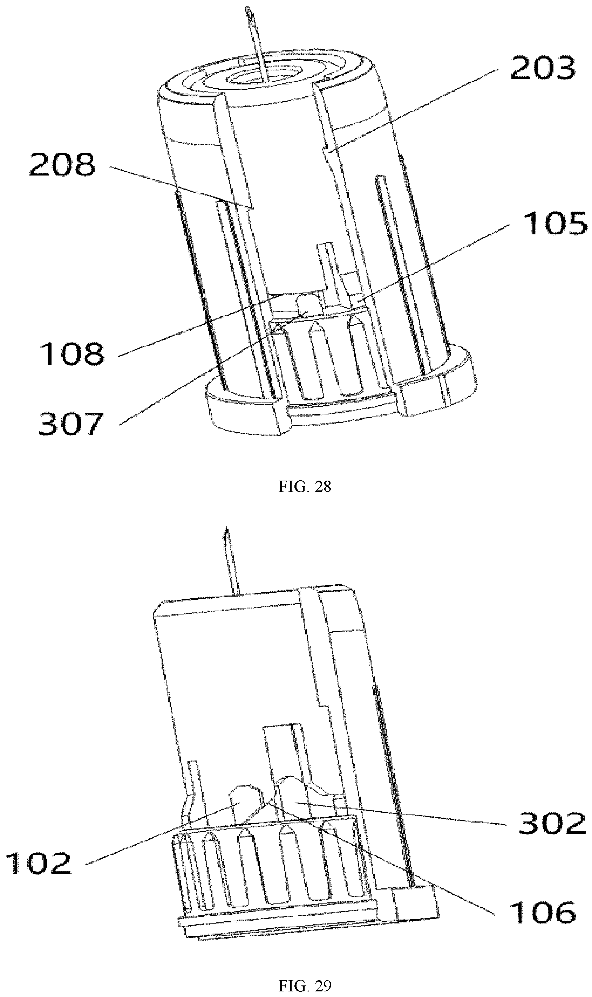

As shown in FIG. 15, FIG. 26 and FIG. 27, based on the movement of the second sheath 10 into the accommodation space R, the inner core 30 moves downward together with the second sheath 10. At this time, the first protrusion 307 is out of abutment with the first step surface 208, and after reaching the end of the guide rib 205, the guide engagement part 3022 moves along the sheath guide part 106, so that the inner core 30 rotates. After the second sheath 10 continues to move downward, the first protrusion 307 abuts against the second step surface 108. Subsequently, the second sheath 10 moves downward together with the inner core 30, as shown in FIG. 28. After the external force is released, they move upward together based on the elastic force of the spring.

In an alternative embodiment, as shown in FIG. 13, the inner core 30 is not provided with the first protrusion 307. At the initial installation position, under the action of the spring 50, the sheath guide part 106 is in contact and engaged with the guide engagement part 3022, the second guide block 302 is in contact with and blocked by the guide rib 205, and the elastic arm 105 is abutted against and in contact with the wedge-shaped structure 203, so that the first sheath 20 and the second sheath 10 are kept in a static state relative to the inner core 30.

As shown in FIG. 6, the second sheath 10 is provided with a first guide groove 104 extending in the axial direction, and the top end of the first guide groove 104 has a second guide surface 1041.

In an alternative embodiment, as shown in FIG. 7, the second guide block 302 has a step engagement surface 3021 at the top of the second guide block 302. Alternatively, the top of the second guide block 302 may not be provided with a step engagement surface 3021.

Based on the movement of the second sheath 10 into the accommodation space R, the inner core 30 moves downward together with the second sheath 10 until the end face of the second guide block 302 reaches the end face of the guide rib 205, and the guide rib 205 releases the restriction on the second guide block 302 in the circumferential direction. Then the guide engagement part 3022 slides relatively along the sheath guide part 106, so that the inner core 30 rotates circumferentially, and the second guide block 302 enters the first guide groove 104 until the top end of the second guide block 302 contacts the second guide surface 1041 of the first guide groove 104. Subsequently, the second sheath 10 and the inner core 30 move downward together under the action of external force. After the external force is released, the second sheath 10 and the inner core 30 move upward together based on the elastic force of the spring.

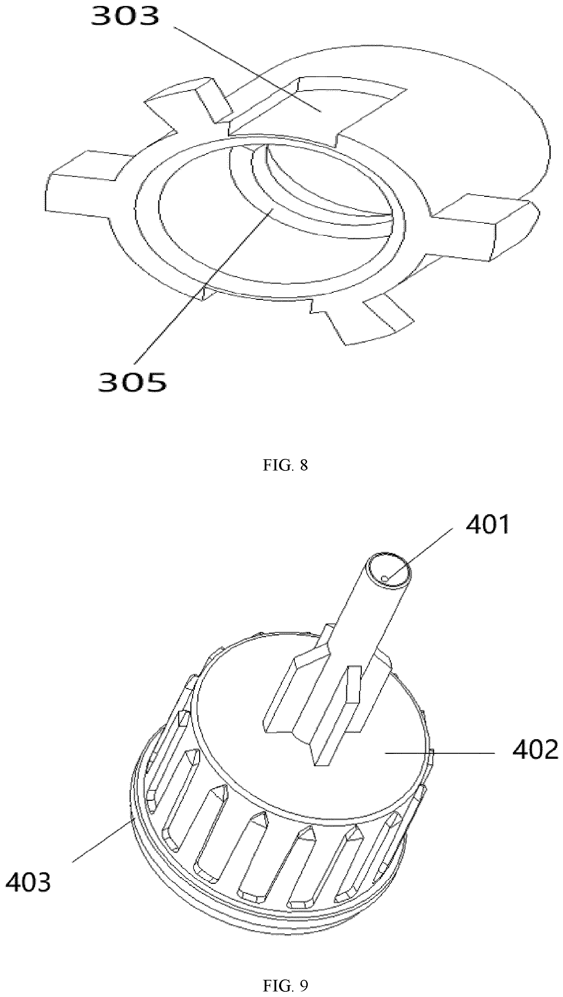

As shown in FIGS. 7 and 8, the outer wall of the inner core 30 has a first concave surface 303, which is a groove extending from the outer wall surface to the inner wall surface.

As shown in FIG. 24, at the initial installation position, that is, at the first circumferential position of the inner core 30, the wedge-shaped structure 203 of the first sheath 20, the elastic arm 105 and the outer wall surface of the inner core 30 are mutually engaged, the inner side surface of the elastic arm 105 is in contact with the outer wall surface of the inner core 30, and the outer side surface of the elastic arm 105 is in contact with the inner side surface of the wedge-shaped structure 203, so that the elastic arm 105 cannot be elastically deformed in the radial direction.

As shown in FIGS. 20, 21 and 26, based on the rotation of the inner core 30 in the circumferential direction, the inner core 30 is in the second circumferential position. At this time, the first concave surface 303 rotates to the position opposite to the elastic arm 105 of the second sheath 10, that is, the first concave surface 303 is located at the side of the elastic arm 105 near the needle tube 60. At this time, the first concave surface 303 provides a space for the elastic arm 105 to elastically deform radially inward.

After the external force is released, the second sheath 10 and the inner core 30 slide upward under the action of the spring 50. When the elastic arm 105 of the second sheath 10 contacts the wedge-shaped structure 203 of the first sheath 20, the first concave surface 303 of the inner core 30 is in the same radial direction with the elastic arm 105, and there is a space between the first concave surface 303 and the elastic arm 105 of the second sheath 10. Therefore, under the action of the spring force, the elastic arm 105 of the second sheath 10 is deformed in the direction of the central axis, and then slides over the wedge-shaped structure 203 of the second sheath 20; after sliding over the he wedge-shaped structure 203, the elastic arm 105 of the second sheath 10 deforms in the direction opposite to the central axis and returns to the natural state.

Subsequently, the second sheath 10 and the inner core 30 continue to move out of the accommodation space R under the action of the spring 50 until the top of the first guide block 102 of the second sheath 10 contacts the top guide surface of the guide groove of the first sheath 20 and stops moving.

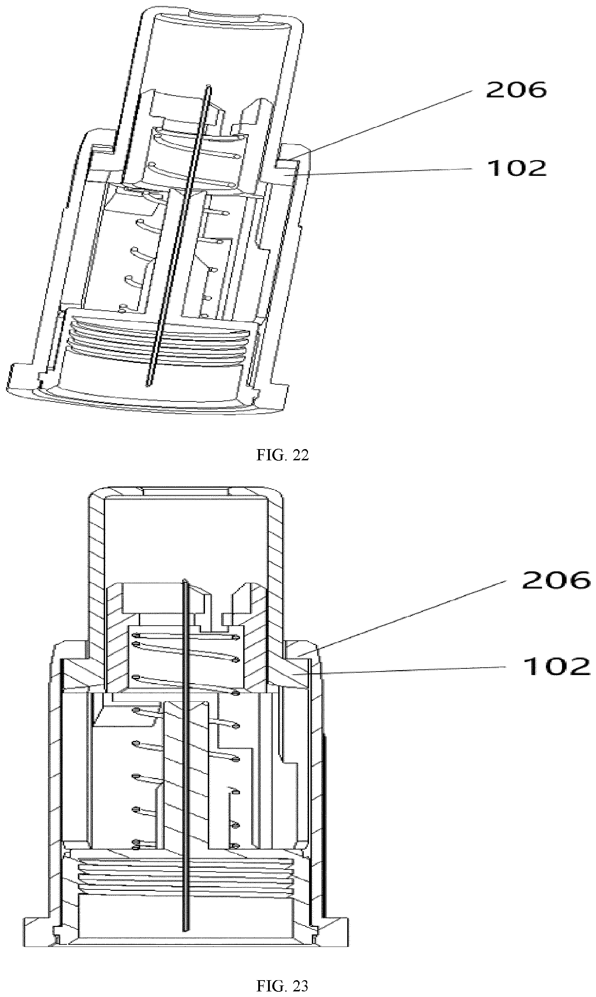

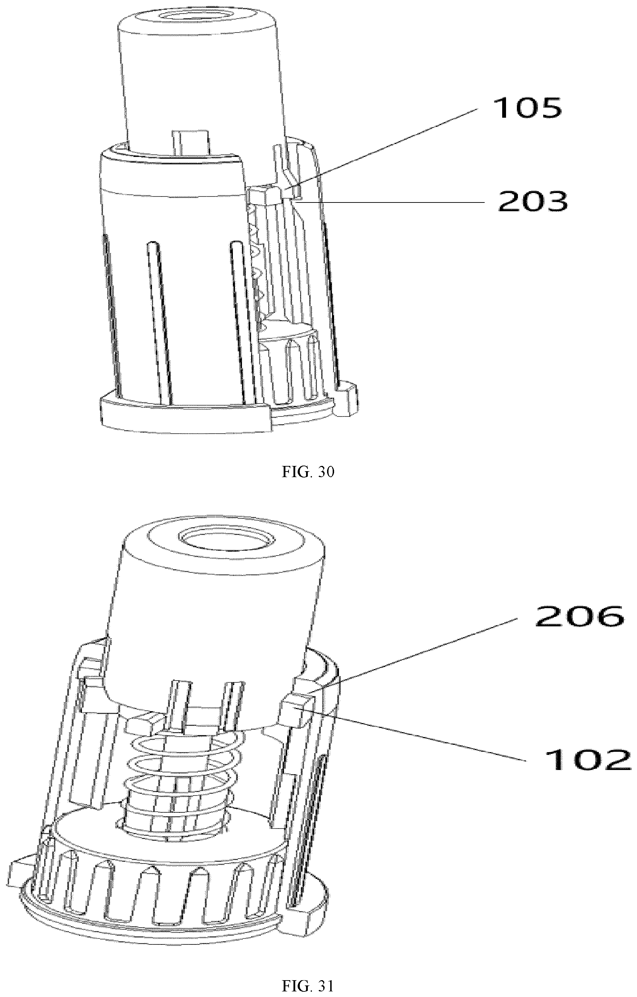

In an alternative embodiment, as shown in FIGS. 22, 23 and 31, the second sheath 10 and the inner core 30 continue to move out of the accommodation space R until the top of the first guide block 102 contacts the first guide surface 206 of the second guide groove 202 of the first sheath 20 and stops moving.

Since at the second circumferential position, after the elastic arm 105 slides over the wedge-shaped structure 203, the bottom end of the elastic arm 105 can abut against the top surface of the wedge-shaped structure 203, so that if the second sheath 10 is pressed down again, the elastic arm 105 of the second sheath 10 is blocked by the wedge-shaped structure 203 from continuously pressing, and the needle tube 60 cannot be exposed from the second opening 107 of the second sheath again. Therefore, the injection needle disclosed by the invention can not be reused after use, and the situation that the needle is easy to injure others by mistake and cause cross infection is avoided.

In an alternative embodiment, as shown in FIG. 4, one end of the first sheath 20 is provided with a base, and the inner wall of the base is provided with transverse grooves 204 arranged circumferentially.

As shown in FIG. 9, one end of the needle seat 40 is provided with transverse convex ribs 403, which are arranged around the outer wall of the needle seat 40 in the circumferential direction. When the disposable injection needle is assembled, the transverse convex ribs 403 are engaged with the transverse grooves 204 to fix the needle seat 40 and the first sheath 20 in the axial position. The center of the needle seat 40 is also provided with a needle seat surface 402 and a needle seat hole 401 through which the needle tube 60 passes.

In an alternative embodiment, before the injection needle of the present disclosure is used, the first end face 306 of the inner core 30 may be higher or lower than the second end face 207 of the first sheath 20 in the axial direction; after use, the first end surface 306 of the inner core 30 may be higher or lower than the second end surface 207 of the first sheath 20 in the axial direction.

In an alternative embodiment, the height of the first end face 306 of the inner core 30 in the axial direction is lower than that of the second end face 207 of the first sheath 20 before the injection needle of the present disclosure is used, that is, at the installation position of the first sheath 20 and the inner core 30; after the injection needle is used, the height of the first end face 306 of the inner core 30 in the axial direction is higher than that of the second end face 207 of the first sheath 20. When the material of the second sheath 10 is transparent or translucent, this difference can be used to distinguish whether the injection needle product of the present disclosure is before use or after use.

With reference to the above structure, the operation process of the disposable injection needle of the exemplary embodiment of the present disclosure will be described below.

First, as shown in FIG. 10, the other end of the second sheath 10 is allowed to pass through the first opening 201 of the first sheath 20, and the first guide block 102 of the second sheath 10 is engaged with the second guide groove 202 of the first sheath 20, so that the second sheath 20 can only move axially relative to the first sheath 10, but cannot rotate circumferentially.

Then, as shown in FIGS. 2, 11, 12 and 14, the inner core 30 is placed in the cavity of the second sheath 20, and the spring 50 is placed in the inner core 30. Under the action of the spring 50, the guide engagement part 3022 of the inner core 30 contacts the sheath guide part 106 of the second sheath 10, and the first protrusion 307 of the inner core 30 contacts the first step surface 208 of the first sheath 20, as shown in FIGS. 24 and 25.

At this time, as shown in FIGS. 11 and 24, the wedge-shaped structure 203 of the first sheath 20 restricts the elastic arm 105 of the first sheath 10 from moving to the other end, so that the second sheath 10 is in the limited position. One end of the spring 50 is in contact with the first blocking surface 305 of the inner core 30, and the other end of the spring 50 is in contact with the needle seat surface 402 of the needle seat 40.

The needle tube 60 passes through the needle seat hole 401 and is fixed in the needle seat hole 401 by an adhesive. The needle seat 40, the spring 50 and the needle tube 60 are assembled in the cavity of the first sheath 20, and the transverse convex ribs 403 of the needle seat 40 are engaged with the transverse grooves 204 of the first sheath 20, so that the positions of the needle seat 40 and the first sheath 20 are relatively fixed.

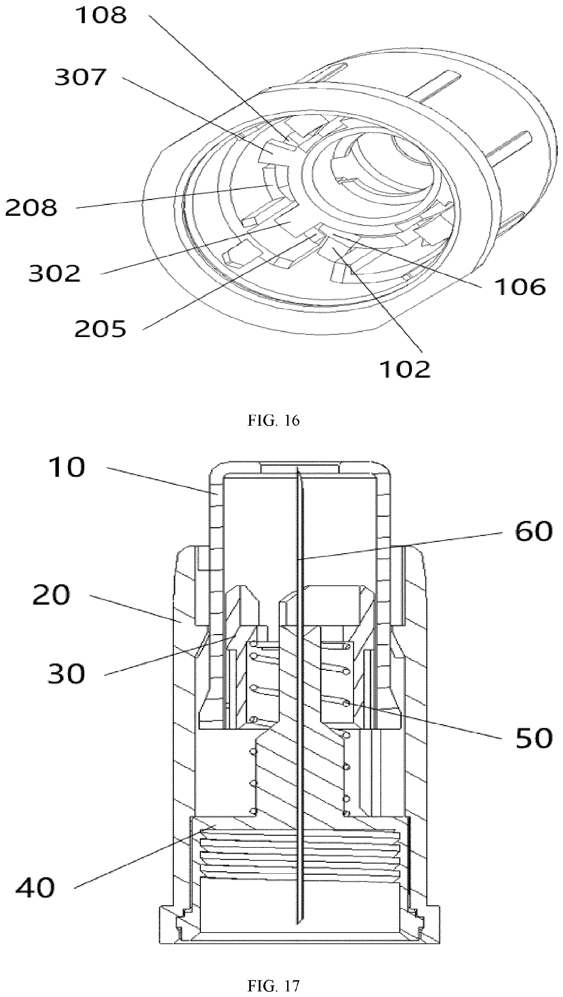

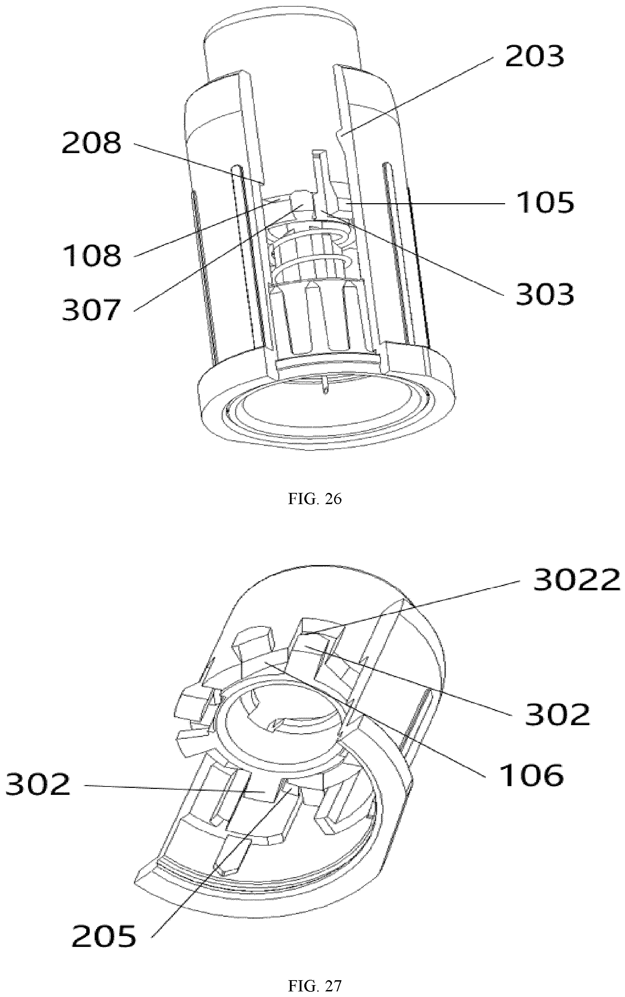

When in use, as shown in FIG. 16, FIG. 17 and FIG. 27, the second sheath 10 moves toward the end of the needle seat 40 under the action of external force until the second guide block 302 breaks away from the obstruction of the guide rib 205 in the circumferential direction. Under the action of the spring 50, the guide engagement part 3022 slides relative to the sheath guide part 106 of the second sheath 10, so that the inner core 30 rotates in the circumferential direction until the first protrusion 307 contacts the second step surface 108 of the second sheath 10. As shown in FIG. 26, the first protrusion 307 of the inner core 30 is separated from the first step surface 208 of the first sheath 20 based on relative rotation and axial movement, and the first concave surface 303 of the inner core 30 rotates to the position of the elastic arm 105 of the second sheath 10.

Under the action of external force, the second sheath 10 continues to move to the end of the needle seat 40 until the second sheath 10 or the inner core 30 contacts the needle seat 40, and the second sheath 10 stops moving. During the axial movement of the second sheath 10, the needle tube 60 is exposed from the second opening 107 of the second sheath 10, as shown in FIGS. 18, 19, 28 and 29.

After the external force is released, as shown in FIG. 20, FIG. 21, FIG. 30 and FIG. 31, the second sheath 10 and the inner core 30 slide upward away from the needle seat 40 under the action of the spring 50. When the elastic arm 105 of the second sheath 10 contacts the wedge-shaped structure 203 of the first sheath 20, since the outer wall surface of the inner core 30 and the elastic arm 105 of the second sheath 10 have been misaligned, the first concave surface 303 is located at the opposite position of the elastic arm 105 of the second sheath 10. At this time, due to the space between the first concave surface 303 and the elastic arm 105 of the second sheath 10, the elastic arm 105 of the second sheath 10 is deformed in the direction of the central axis under the action of the spring force and slides over the wedge-shaped structure 203 of the first sheath 20. After sliding over the wedge-shaped structure 203 of the first sheath 20, the elastic arm 105 of the second sheath 10 deforms in the direction opposite to the central axis and returns to the natural state.

As shown in FIGS. 22, 23 and 31, the second sheath 10 and the inner core 30 continue to move under the action of the spring until the first guide block 102 of the second sheath 10 stops moving when it contacts the first guide surface 206 of the first sheath 20.

When the second sheath 10 is pressed down again, since the elastic arm 105 of the second sheath 10 interferes with the wedge-shaped structure 203 of the first sheath 20, that is, the top surface of the wedge-shaped structure 203 can abut against the bottom end of the elastic arm 105, which prevents the elastic arm 105 from moving down, it cannot be pressed down, as shown in FIGS. 20, 21 and 30. The needle tube 60 cannot be exposed from the second opening 107 of the second sheath 10 again, and thus it cannot be used again.

It should be noted that the above technical solutions can be combined in any logical situation, which shall fall within the protection scope of this disclosure. In this specification, relational terms such as "first" and "second" are only used to distinguish one entity or operation from another entity or operation without necessarily requiring or implying any such actual relationship or order between these entities or operations. Moreover, the terms "including", "comprising" or any other variation thereof are intended to cover non-exclusive inclusion, so that a process, method, article or equipment including a series of elements includes not only those elements, but also other elements not explicitly listed or elements inherent to such process, method, article or equipment. Without further restrictions, an element defined by the phrase "including one" does not exclude the existence of other identical elements in the process, method, article or equipment including the element.

Each embodiment in this specification is described in a related way, and only the same and similar parts between the embodiments can be referred to each other, and each embodiment focuses on the differences from other embodiments.

## Claims

1. A disposable injection needle, comprising:
a needle seat (40), wherein the needle seat is provided with a needle tube (60) extending axially therethrough;
a first sheath (20), wherein one end of the first sheath is connected to one end of the needle seat to form an accommodating space between the first sheath and the needle seat, and the other end of the first sheath has a first opening (201);
a second sheath (10), wherein one end of the second sheath is located in the accommodating space, and the other end of the second sheath has a second opening (107) and protruding from the first opening; a part of the second sheath is located in the accommodating space in an axially slidable manner;
an inner core (30), wherein the other end of the inner core is provided with a third opening (304), and the needle tube axially extends through the third opening and is adapted for being exposed via the second opening; and
a spring (50);
wherein:
the one end of the second sheath is engaged with the inner core by sleeving, and is adapted for retracting towards the accommodating space to expose the needle tube against an elastic force of the spring based on an external force;
the spring is adapted for providing an elastic force to make the second sheath and the inner core move axially towards the outside of the accommodating space together;
an inner wall of the first sheath is provided with a first engagement structure, and the second sheath is provided with a second engagement structure engaged with the first engagement structure;
based on the movement of the second sheath into the accommodating space, the inner core rotates from a first circumferential position to a second circumferential position, and at the second circumferential position, the inner core is adapted to be abutted by the spring and move in a direction away from the accommodating space to push the second sheath; and
at the second circumferential position, the second engagement structure is adapted to pass over the first engagement structure in a direction away from the accommodating space, and the first engagement structure is adapted to prevent the second engagement structure that has passed over the first engagement structure from moving further in a direction toward the inside of the accommodating space.

2. The disposable injection needle according to claim 1, wherein:
the first engagement structure is a radially inward convex structure, and the second engagement structure is an elastic arm (105);
at the first circumferential position, the convex structure is adapted to be arranged opposite to the elastic arm; at the second circumferential position, an end of the elastic arm is adapted for abutting against a top of the convex structure.

3. The disposable injection needle according to claim 2, wherein:
the convex structure is a wedge-shaped structure (203);
at the first circumferential position, an outer side surface of the elastic arm is adapted to be arranged opposite to a side surface of the wedge-shaped structure; at the second circumferential position, the end of the elastic arm is adapted for abutting against a top surface of the wedge-shaped structure.

4. The disposable injection needle according to claim 2 or 3, wherein:
an outer wall of the inner core has a first concave surface (303), which is adapted to be arranged opposite to the elastic arm to provide an elastic deformation space for the elastic arm based on the rotation of the inner core in the circumferential direction.

5. The disposable injection needle according to any one of claim 1-4, wherein:
one end of the second sheath is provided with a sheath guide part (106), and one end of the inner core is provided with a guide engagement part (3022), which is engaged with the guide engagement part at an installation position of the second sheath and the inner core; and
based on the movement of the second sheath into the accommodating space, the guide engagement part is adapted to move along the sheath guide part to guide the inner core to rotate in the circumferential direction and is adapted to move out of engagement with the sheath guide part.

6. The disposable injection needle according to claim 5, wherein:
the inner wall of the first sheath is provided with a guide rib (205) extending in the axial direction, and a second guide groove (202) and a third guide groove (209) extending in the axial direction are respectively provided on the two sides of the guide rib;
the inner core is provided with a second guide block (302) extending radially outward, and the second guide block is provided with the guide engagement part (3022);
at the installation position of the second sheath and the inner core, the second guide block (302) is located in the second guide groove (202); based on the movement of the second sheath into the accommodating space and the rotation of the inner core in the circumferential direction, the second guide block (302) passes over the guide rib (205) and enters the third guide groove.

7. The disposable injection needle according to claim 6, wherein:
the inner wall of the first sheath is provided with a guide groove extending in the axial direction, and an outer wall of the second sheath is provided with a guide block extending radially outward; and the guide block is adapted for moving in the axial direction along the guide groove;
optionally, the guide groove is the second guide groove (202);
further optionally, the guide block is a first guide block (102), which is located outside the wall where the sheath guide part (106) is located, and an end of the first guide block is an inclined plane with a same inclination angle as the sheath guide part.

8. The disposable injection needle according to claim 6, wherein:
the ends of the sheath guide part (106), the guide engagement part (3022) and the guide rib (205) are inclined planes with a same inclination angle.

9. The disposable injection needle according to any one of claim 1-8, wherein:
the inner core is provided with a first protrusion (307) extending radially outward, and the inner wall of the first sheath is provided with a first step surface (208);
at the installation position of the first sheath and the inner core, the first protrusion is adapted for abutting against the first step surface.

10. The disposable injection needle according to claim 9, wherein:
one end of the second sheath is provided with a second step surface (108);
the inner core rotates in the circumferential direction, so that the first protrusion (307) breaks away from the abutment with the first step surface (208) and abuts against the second step surface (108).

11. The disposable injection needle according to any one of claim 1-8, wherein:
the second sheath is provided with a first guide groove (104) extending in the axial direction, and the guide engagement part is adapted to enter the first guide groove based on the rotation of the inner core in the circumferential direction.

12. The disposable injection needle according to claim 11, wherein:
the inner core is provided with a second guide block (302) extending radially outward, and the second guide block is provided with the guide engagement part (3022);
an end of the first guide groove (104) is provided with a second guide surface (1041);
based on the rotation of the inner core in the circumferential direction, the second guide block is adapted to enter the first guide groove until the second guide block abuts against the second guide surface.

13. The disposable injection needle according to any one of claim 1-8, wherein:
an annular rib is arranged on an inner wall of the inner core, a first blocking surface (305) is formed on the annular rib, and one end of the spring abuts against the first blocking surface.

14. The disposable injection needle according to any one of claim 1-8, wherein:
at the installation position of the first sheath and the inner core, a height of a first end face of the inner core in the axial direction is lower than that of a second end face of the first sheath; and
after the injection needle is used, the height of the first end face of the inner core in the axial direction is higher than that of the second end face of the first sheath.

15. The disposable injection needle according to any one of claim 1-8, wherein:
the one end of the first sheath is provided with a base, and an inner wall of the base is provided with transverse grooves (204) arranged circumferentially; and
the one end of the needle seat is provided with transverse convex ribs (403), and the transverse convex ribs are circumferentially arranged on an outer wall of the needle seat; and the transverse convex ribs are engaged in the transverse grooves.
